Europäisches Patentamt

(19) European Patent Office

Office européen des brevets

(11) Publication number: **0 041 569**
**B1**

(12) **EUROPEAN PATENT SPECIFICATION**

(45) Date of publication of patent specification: **31.10.84**

(51) Int. Cl.³: **A 41 B 13/02**

(21) Application number: **81900185.0**

(22) Date of filing: **09.12.80**

(86) International application number:
**PCT/US80/01662**

(87) International publication number:
**WO 81/01643 25.06.81 Gazette 81/15**

(54) **DIAPER.**

(30) Priority: **10.12.79 US 101776**

(43) Date of publication of application:
**16.12.81 Bulletin 81/50**

(45) Publication of the grant of the patent:
**31.10.84 Bulletin 84/44**

(84) Designated Contracting States:
**DE FR GB NL SE**

(56) References cited:
**GB-A- 748 135**
**US-A-2 023 253**
**US-A-2 750 944**
**US-A-3 172 817**
**US-A-3 340 875**
**US-A-3 381 688**
**US-A-3 528 421**
**US-A-3 707 148**
**US-A-3 832 327**
**US-A-3 939 838**

(73) Proprietor: **MOLECULAR SIEVE SYSTEMS, INC.**
**26 Cedar Street**
**Worcester, MA 01606 (US)**

(72) Inventor: **DODWELL, Glenn W.**
**26 Cedar Street**
**Worcester, MA 01606 (US)**

(74) Representative: **Sommerville, John Henry et al**
**SOMMERVILLE & RUSHTON 11 Holywell Hill**
**St. Albans Hertfordshire, AL1 1EZ (GB)**

Courier Press, Leamington Spa, England.

## Description

Conventional, commercially available diapers of the washable type are simply cotton fabric which, except for the inherent water adsorption of the cotton have no special characteristics to absorb, remove, or otherwise treat or condition the urine and feces contained therein during use.

Commercially available disposable diapers have a layer next to the skin which is relatively non-water absorbing but is porous to water, and an inner water absorbent cellulosic fibrous web of fillings, with an outer more-or-less water-impervious layer.

Several patents have been issued which relate attempts to alleviate the irritation caused by the excreta on the wearer's skin. In particular US—A—2,643,969 teaches the use of complex organic bacteriostats or bacteriocides to stop or control the action of bacteria which generate ammonia from the nitrogenous waste material, and describes prior use of bichloride of mercury and boric acid as rinses to provide the diaper material with antiseptic properties. US—A—3,922,723 describes a chemical treatment for cotton-wearing apparel to give it anion exchange properties for control of body odor. There is also reference in the patent to chemically attracting anion exchange resins to fabrics, and (in column 10) it is suggested that cation exchange cottons can be produced by chemical treatment, and that such treatment may be employed in the garments described in the patent. At column 8 of the patent there is a reference to use of the invention in many kinds of garment, including the diaper. US—A—2,690,415, describes the use of carbon black and silica gel, adhesively secured to the yarns of a fabric for producing an odor eliminating blanket, bandage, or catamenial pad. US—A—3,935,363 teaches bentonite as a liquid absorbent in diapers.

The patent and other literature teaches that ammonia present, in the waste in a diaper is a cause of diaper rash, and that such disease of the skin in infants can be a serious medical matter which can in extreme cases result in death. US—A—3,567,820, teaches incorporating organic cation exchange resins in an ointment for application to skin which is afflicted with ammonia dermatitis, or diaper rash. Such resin is present to remove ammonium ions present, or produced by bacterial action in, the diaper contents.

### Summary of the Invention

In the present invention, ammonia (and other toxic or potentially toxic nitrogenous irritants) are removed from the waste matter in the diaper by the incorporation into the diaper of an inorganic aluminosilicate zeolite ammonium ion exchange material. Materials useful in the present invention have a high and a selective capacity for ammonium ion exchange, and have additional sorptive capacity for polar molecules in the intracrystalline pores of the zeolite. Such inorganic aluminosilicate zeolites may be natural or synthetic. Synthetic examples are zeolite F, zeolite W, zeolite A, synthetic faujasites (zeolites X and Y), synthetic mordenite, synthetic clinoptilolite, synthetic gismondine-types, and synthetic phillipsite-types. Examples of the natural zeolites useful in the invention are erionite, ferrierite, chabazite, phillipsite, mordenite, and clinoptilolite. Where it is particularly desirable, a portion of the zeolite may be converted partially or completely to another alkaline earth or alkali ion exchange form such as Na.

Additional water removal can be achieved by incorporating conventional sorbents such as silica gel in the diaper. Additional capacity for removal of ammonia or other toxic substance in the waste can be achieved by incorporation of activated carbon, amorphous permutite type aluminosilicates, crystalline aluminosilicates, or clay minerals such as kaolinite, bentonite, sepiolite, and attapulgite.

The invention can be applied to conventional cloth diapers and is especially adaptable to disposable, one use diapers.

For conventional cloth diapers, a soft, flexible, water-permeable layer retentively incorporating the ammonium exchange material and any other material such as clay or clay minerals and buffers, is applied to the cloth diapers, or such layer could be applied to the outside or the inside of the diaper in any convenient manner, by pinning or taping, or loosely inserting. A sprinkling of powder of the appropriate zeolite-clay combination can be applied to the surface of the inner layer of the diaper.

In case of disposable diapers, the ammonium exchange material is incorporated in the cellulosic fibrous layer between the porous, permeable sheet adapted to be worn next to the skin of the user, and the normally water-impermeable outer layer of the diaper. If desired, the outer layer need not be impermeable to water, its function being primarily to contain the zeolite and any other sorbent or active materials in the diaper.

The form of the zeolite and of the silica gel, amorphous or activated carbon and clay minerals will normally be powder or finely divided particulate. Such materials can be effectively retained among the fibers in the interlayer of the diaper. If desired, the zeolite and other particulate materials may be retained in relatively small pouches formed between the inner and outer layers of the diaper, whereby a quilted effect is achieved, as by employing heat bonding of the boundaries of the pouches when at least one of the layers is thermoplastic, or by employing conventional adhesives or by mechanical stitching.

In cases where sodium exchanged zeolites are used to take advantage of the enhanced selectivity for ammonium ion exchange of certain such zeolites for example, mordenite and clinoptilolite. It is desirable to control the pH by the use of buffers. For example, an addition of powdered disodium hydrogen phosphate in such proportion to yield approximately $2.5 \times 10^{-2}$ molar concentration in a wet diaper which will buffer a pH range near 7. Other examples of suitable buffers include the combination of potassium dihydrogen phosphate and disodium hydrogen phosphate powders to yield equimolar concentrations of $3 \times 10^{-2}$ molar in the urine of the wet diaper. Other buffers, for example, boric acid buffers, which will contribute to the control of the growth of bacteria, mold and yeast, can be employed to yield the desired pH. Other buffers yielding a pH range in the urine of the wet diaper of 6 to 8 may also be used.

Description of the Specific Embodiment of Invention

For each square foot (929 square centimeters) of operational diaper area, to significantly reduce irritation from urine and feces (primarily caused by nitrogenous compounds) in effective formulation is: 60 grams of naturally occurring clinoptilolite, in the natural ion exchange form, or preferably, at least, partially or completely sodium exchanged; 35 grams of silica or silica-alumina gel; and 10 grams of kaolinite, 15 grams bentonite, and 3 grams activated carbon. The ingredients are evenly dispersed within the fibrous interlayer of a conventional disposable diaper. Most preferably it is desirable to add a buffer to the above ingredients such as to produce a pH in the wet diaper of approximately 7. A suitable average amount for the 1 square foot of operational diaper area is 0.36 grams $Na_2HPO_4$ and 0.30 grams $KH_2PO_4$.

Claims

1. A diaper having a fabric sheet on one side thereof to be worn next to the skin of the user, a fabric layer on the opposite side of said fabric sheet incorporating an effective amount of basic inorganic zeolite cation exchange material capable of preferentially incorporating ammonium ions whereby the ammonium content of excreta in contact with said diaper in use is reduced.

2. A diaper as in Claim 1 in which the zeolite is selected from the group consisting of synthetic zeolites such as zeolite F, zeolite W, zeolite A, synthetic gismondine-types, and/or synthetic or natural mordenite, chabazite, phillipsite, and clinoptilolite, and mixtures thereof.

3. A diaper as in Claim 1 in which the zeolite is sodium mordenite.

4. A diaper as in Claim 1 in which the zeolite is sodium clinoptilolite.

5. A diaper as in Claim 1 in which the zeolite is zeolite F in a potassium form.

6. A diaper as in Claim 1 wherein the cation exchange material is a permutite type crystallographically amorphous aluminosilicate.

7. A diaper as in Claim 1 including a buffer.

Patentansprüche

1. Windel mit einer Gewebelage an ihrer einen Seite, die der Haut des Benutzers am nächsten au liegen kommt, mit einer weiteren Gewebelage, die auf der gegenüberliegenden Seite der erstgenannten Gewebelage liegt und eine wirksame Menge eines anorganischen, basischen, zeolithischen Kationen-Austauschmateriales umfaßt, das in der Lage ist, vorzugweise Ammonium-Ionen aufzunehmen, wobei der Ammoniumgehalt der mit der Windel in Berührung gelangenden Exkremente verringert wird.

2. Windel nach Anspruch 1, dadurch gekennzeichnet, daß das Zeolith aus einer Gruppe ausgewählt ist, die aus synthetischen Zeolithen wie Zeolith F, Zeolith W, Zeolith A, synthetischen Gismondin- Typen und/oder synthetischen oder natürlichen Mordeniten, Chabaziten, Phillipsiten, und Clinoptilolien sowie aus Gemischen hieraus besteht.

3. Windel nach Anspruch 1, wobei das Zeolith eine Natrium-Mordenit ist.

4. Windel nach Anspruch 1, wobei das Zeolith eine Natrium-Clinoptilolit ist.

5. Windel nach Anspruch 1, wobei das Zeolith eine Zeolith F in Kaliumform ist.

6. Windel nach Anspruch 1, wobei das Kationen-Austausch-material ein kristallin-amorphes Aluminiumsilikat vom Permutit-Typus ist.

7. Windel nach Anspruch 1 mit einem Puffer.

Revendications

1. Couche présentant d'une côté une feuille de tissu destinée à être portée au contact de la peau de l'usager et, de l'autre côté de ladite feuille de tissu, une surface tissée incorporant en quantité efficace un mátériau zéolithique, inorganique et basique, échangeur de cation, et capable de s'incorporer préférentiellement des ions ammonium, tandis que la concentration en ion ammonium des excréments en contact avec ladite couche est réduite lors de sont utilisation.

2. Couche selon la revendication 1, dans laquelle le matériau zéolithique est choisi dans le groupe constitué par les zéolithiques F, W, A, les types similaires à la gismandine synthétique et/ou les mordénites, chabazites, phillipsites et clinoptilolithes synthétiques et naturelles, et leurs mélanges.

3. Couche selon la revendication 1, dans laquelle la zéolithe, est de la mordénite de sodium.

4. Couche selon la revendication 1, dans laquelle la zéolithe est de la clinoptilolite de sodium.

5. Couche selon la revendication 1, dans laquelle le zéolithe est de la zéolithe F dans la forme potassium.

6. Couche selon la revendication 1, dans laquelle la matériau échangeur de cation est un aminosilicate critallographiquement amorphe, du type permutite.

7. Couche selon la revendication 1 contenant un tampon.